(19) Europäisches Patentamt
European Patent Office
Office européen des brevets

(11) **EP 3 437 625 B1**

(12) **EUROPÄISCHE PATENTSCHRIFT**

(45) Veröffentlichungstag und Bekanntmachung des Hinweises auf die Patenterteilung:
**19.02.2020 Patentblatt 2020/08**

(51) Int Cl.:
*A61K 8/25* *(2006.01)*      *A61K 8/49* *(2006.01)*
*A61K 8/35* *(2006.01)*      *A61Q 17/04* *(2006.01)*

(21) Anmeldenummer: **18181900.4**

(22) Anmeldetag: **05.07.2018**

(54) **SPHÄRISCHE SILIKATE IN KOSMETISCHEN ZUBEREITUNGEN**

SPHERICAL SILICATES IN COSMETIC PREPARATIONS

SILICATES SPHÉRIQUES DANS LES PRÉPARATIONS COSMÉTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.08.2017 DE 102017213230**

(43) Veröffentlichungstag der Anmeldung:
**06.02.2019 Patentblatt 2019/06**

(73) Patentinhaber: **Beiersdorf AG**
**20253 Hamburg (DE)**

(72) Erfinder:
• **Eitrich, Anja**
**25337 Kölln-Reisiek (DE)**
• **Deinhard, Sybil-Marie**
**22307 Hamburg (DE)**
• **Schade, Juliane**
**22529 Hamburg (DE)**

(56) Entgegenhaltungen:
**EP-A1- 3 093 007      WO-A1-2015/150169**
**JP-A- 2011 105 626**

EP 3 437 625 B1

**Beschreibung**

Die vorliegende Erfindung betrifft die Verwendung eines sphärischen Silikats mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101) in kosmetischen Zubereitungen zur Reduzierung der Sand anhaftenden Eigenschaften der Zubereitung, sowie eine kosmetische Zubereitung enthaltend ein sphärisches Silikat mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101) und mindestens einen im UV-A Bereich absorbierenden organischen UV-Filter.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen:
Kosmetische Sonnenschutzmittel weisen unter anderem aufgrund ihres UV-Filtergehaltes meist eine gewisse Klebrigkeit auf, die insbesondere bei der Verwendung am Strand dazu führt, dass es auf eingecremten Hautpartien zur Anhaftung von Sand kommt. Dieses Problem wird umso größer, je mehr UV-Filter eine Zubereitung enthält. Zwar hat es in der Vergangenheit nicht an Versuchen gefehlt, sandabweisende Sonnenschutzmittel zu entwickeln, doch ist dieses Problem besonders bei Zubereitungen mit hohem Lichtschutzfaktor bis heute nicht endgültig zufriedenstellend gelöst.
Es war daher die Aufgabe der vorliegenden Erfindung, ein Sonnenschutzmittel mit reduzierter Sandanhaftung ("sandabweisend") zu entwickeln. Insbesondere sollte ein Sonnenschutzmittel mit hohem Lichtschutzfaktor (SPF 30 und höher) entwickelt werden, welches besonders wenig sandanhaftend ist.

Überraschend gelöst wird die Aufgabe durch die Verwendung eines sphärischen Silikats mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101) in kosmetischen Zubereitungen zur Reduzierung der Sand anhaftenden Eigenschaften der Zubereitung.
Dabei ist diese Verwendung erfindungsgemäß bevorzugt dadurch gekennzeichnet, dass die Zubereitung mindestens einen im UV-A Bereich absorbierenden organischen UV-Filter enthält.
Überraschend wird die Aufgabe ferner gelöst durch eine kosmetische Zubereitung enthaltend a) ein sphärisches Silikat mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101) b) mindestens einen im UV-A Bereich absorbierenden organischen UV-Filter.
Zwar kennt der Fachmann natürlich eine Reihe von kosmetischen Zubereitungen und insbesondere Sonnenschutzmittel, die Silica enthalten, doch weisen diese andere Größen, Formen und Absorptionseigenschaften auf. Wie sich aus den Vergleichsversuchen (siehe unten) jedoch ergibt, ist das erfindungsgemäße Silica besonders geeignet, die Sandanhaftung zu unterdrücken. Darüber hinaus wurde der Einfluss des Silicas und seiner Eigenschaften auf die Sandanhaftung bisher nicht untersucht und beschrieben. So siehe z.B. die EP 3093007 A1.
Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäßen Verwendungen als auch auf die erfindungsgemäße Zubereitung.

Es ist erfindungsgemäß vorteilhaft, wenn das erfindungsgemäße sphärische Silikat in einer Konzentration von 0,5 bis 5% Gewichts-%, bezogen auf das Gesamtgewicht der Zubereitung, enthält. Dabei ist ein Konzentrationsbereich von 1,5 bis 3 Gewichts-%, bezogen auf das Gesamtgewicht der Zubereitung, erfindungsgemäß bevorzugt.

Darüber hinaus ist es erfindungsgemäß von Vorteil, wenn das erfindungsgemäße sphärische Silikat eine spezifische Oberfläche von 600 bis 800 m$^2$/g (Messmethode: BET mit N$_2$) und ein spezifisches Porenvolumen von 1,4 bis 1,8 ml/g (Messmethode: BET mit N$_2$) aufweist.

Als erfindungsgemäßes sphärisches Silikat kann beispielsweise das Produkt "sunsphere H-52" der Firma AGC Si-Tech Co. Ltd. Aus Fukuoka (Japan) eingesetzt werden.

Die erfindungsgemäße Teilchengröße wurde mit der "Coulter Counter (d50)"-Methode bestimmt.

Die Messung der Ölabsorption, gemessen nach dem japanischen Industriestandard K-5101, erfolgt mit kochendem Leinsamenöl (boiled linseed oil, spezifiziert im japanischen Standard K 5421).

Erfindungsgemäß bevorzugt werden der oder die im UV-A Bereich absorbierenden organischen UV-Filter gewählt werden aus der Gruppe der Verbindungen 4-(tert.-Butyl)-4'-methoxydiben-zoylmethan, Hexyl 2-[4-(diethylamino)-2-hydroxybenzoyl]benzoate (INCI: Diethylamino hydroxybenzoyl hexyl benzoate) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

Erfindungsgemäß besonders bevorzugt ist dabei eine Kombination aus 4-(tert.-Butyl)-4'-methoxydibenzoylmethan und 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

Die erfindungsgemäß vorteilhaften Einsatzkonzentrationen betragen für 4-(tert.-Butyl)-4'-methoxydibenzoylmethan von 1 bis 5 Gewichts-%, für Hexyl 2-[4-(diethylamino)-2-hydroxybenzoyl]benzoate (INCI: Diethylamino hydroxybenzoyl hexyl benzoate) von 1 bis 5 Gewichts-% und für 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) von 1 bis 5 Gewichts-%, jeweils bezogen auf das Gesamtgewicht der Zubereitung.

Darüber hinaus kann die erfindungsgemäße Zubereitung vorteilhaft einen oder mehrere weitere UV-Filter gewählt aus der Gruppe der Verbindungen Salicylsäure-2-ethylhexylester (INCI Octyl Salicylate), 3,3,5-Trimethylcyclohexyl 2-hydroxybenzoat (INCI: Homosalate), 2,4,6-Tris-[anilino-(p-carbo-2'-ethyl-1'-hexyloxy)]-1,3,5-triazin (INCI: Ethylhexyl Triazone) und/oder 2-Phenylbenzimidazol-5-sulfonsäuresalze, enthalten.

Vorteilhafte Ausführungsformen der vorliegenden Erfindung sind ferner dadurch gekennzeichnet, dass die Zubereitung frei ist von 3-(4-Methylbenzyliden)-campher, 2-Hydroxy-4-methoxybenzophenon (INCI: Oxybenzon), Ethylhexyl-2-cyano-3,3-diphenylacrylat (INCI: Octocrylen), Parabenen, Methylisothiazolinon, Chlormethyl-isothiazolinon und DMDM-Hydantoin.

Insbesondere ist es erfindungsgemäß von Vorteil, wenn die Zubereitung frei ist von 4-Methoxyzimtsäure-2-ethylhexylester (INCI Octylmethoxycinnamate).

Darüber hinaus ist es erfindungsgemäß von Vorteil, wenn Verwendung die erfindungsgemäße Zubereitung keine Polyethylenglycolether oder Polyethylenglycolester enthält.

Die erfindungsgemäße Zubereitung kann erfindungsgemäß in unterschiedlichen Formen vorliegen. Die erfindungsgemäß bevorzugten Formen sind dadurch gekennzeichnet, dass die Zubereitung in Form einer O/W-Emulsion vorliegt.

Liegt die erfindungsgemäße Zubereitung in Form einer O/W-Emulsion vor, so ist sie erfindungsgemäß vorteilhaft dadurch gekennzeichnet, dass die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Kaliumcetylphosphat, Polyglyceryl-10 Stearate , Natriumstearylglutamat, enthält.

Es ist erfindungsgemäß bevorzugt, wenn die Zubereitung Natriumstearylglutamat als Emulgator enthält.

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

Darüber hinaus sind die erfindungsgemäß vorteilhaften Ausführungsformen der vorliegenden Erfindung dadurch gekennzeichnet, dass die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

Erfindungsgemäß besonders vorteilhafte Ausführungsformen der vorliegenden Erfindung sind auch dadurch gekennzeichnet, dass die Zubereitung Ethanol, Phenoxyethanol und/oder Ethylhexylglycerin enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen. Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon. Es ist erfindungsgemäß bevorzugt, wenn die erfindungsgemäße Zubereitung Tapiocastärke enthält. Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat, Butylene Glycol Dicaprylat/Dicaprat und/oder C12-C15 Alkylbenzoat enthält. Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Es ist dabei erfindungsgemäß bevorzugt, wenn die Zubereitung Xanthangummi und/oder quervernetztes Acrylat/C10-C30 Alkylacrylat Polymer enthält.

Ein Gehalt an Glycerin von mindestens 1 Gewicht-%, bezogen auf das Gesamtgewicht der Zubereitung, ist erfindungsgemäß besonders vorteilhaft.

Es ist ferner vorteilhaft im Sinne der vorliegenden Erfindung, wenn die erfindungsgemäße Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin enthält.

Darüber hinaus können die erfindungsgemäßen Zubereitungen die für kosmetische Sonnenschutzmittel üblichen Inhaltsstoffe in den üblichen Einsatzkonzentrationen enthalten.

**Vergleichsversuch**

Der erfindungsgemäße Effekt konnte mit dem folgenden Vergleichsversuch belegt werden. Es handelt sich dabei um identische Rezepturen, die sich allein in der Art des eingesetzten Silicas unterscheiden.

Es wurden die folgenden Rezepturen hergestellt:

| INCI | MATTSUN 3017 m [%] | MATTSUN 3019 m [%] | MATTSUN 3021 m [%] | MATTSUN 3040 m [%] | MATTSUN 3041 m [%] |
|---|---|---|---|---|---|
| Aqua | 60,81 | 60,79 | 60,79 | 60,81 | 60,81 |
| Tocopheryl Acetate + Glycyrrhiza Inflata Root Extract | 0,13 | 0,13 | 0,13 | 0,13 | 0,13 |
| Dibutyl Adipate + C12-15 Alkyl Benzoate + Butylene Glycol Dicaprylate/Dicaprate | 8,00 | 8,00 | 8,00 | 8,00 | 8,00 |

(fortgesetzt)

| INCI | MATTSUN 3017 m [%] | MATTSUN 3019 m [%] | MATTSUN 3021 m [%] | MATTSUN 3040 m [%] | MATTSUN 3041 m [%] |
|---|---|---|---|---|---|
| Cetearyl Alcohol + Behenyl Alcohol | 2,00 | 2,00 | 2,00 | 2,00 | 2,00 |
| Aqua + Trisodium EDTA | 1,00 | 1,00 | 1,00 | 1,00 | 1,00 |
| Dimethicone + Cyclomethicone | 2,80 | 2,80 | 2,80 | 2,80 | 2,80 |
| Butyl Methoxydibenzoylmethane | 3,00 | 3,00 | 3,00 | 3,00 | 3,00 |
| Phenylbenzimidazole Sulfonic Acid | 2,00 | 2,00 | 2,00 | 2,00 | 2,00 |
| Aqua + Sodium Hydroxide | 0,92 | 0,94 | 0,94 | 0,92 | 0,92 |
| Glycerin + Alcohol Denat. + Aqua | 5,00 | 5,00 | 5,00 | 5,00 | 5,00 |
| Ethylhexyl Salicylate + Ethylhexyl Triazone | 5,00 | 5,00 | 5,00 | 5,00 | 5,00 |
| Xanthan Gum + Acrylates/C10-30 Alkyl Acrylate Crosspolymer + Carbomer | 0,35 | 0,35 | 0,35 | 0,35 | 0,35 |
| Ethylhexylglycerin + Phenoxyethanol + Methylpropandiol | 1,90 | 1,90 | 1,90 | 1,90 | 1,90 |
| Bis-Ethylhexyloxyphenol Methoxyphenyl Triazine | 2,50 | 2,50 | 2,50 | 2,50 | 2,50 |
| Tapioca Starch + Aqua | 2,00 | 2,00 | 2,00 | 2,00 | 2,00 |
| Sodium Stearoyl Glutamate | 0,30 | 0,30 | 0,30 | 0,30 | 0,30 |
| Parfum | 0,30 | 0,30 | 0,30 | 0,30 | 0,30 |
| **Silica (Sunsphere H-52)** | **2,00** | | | | |
| **Silica (Sunsphere H-121)** | | | | | **2,00** |
| **Silica (Sunsil-130)** | | | **2,00** | | |
| **Silica Micro Bead BA-1)** | | | | **2,00** | |
| **Silica (Valvance Touch 210)** | | **2,00** | | | |
| | | | | | |
| **Summen:** | **100,00** | **100,00** | **100,00** | **100,00** | **100,00** |

Silica (Sunsphere H-52): sphärisch, mittlere Teilchengröße: 5 µm, Ölabsorption: 300 mg, erfindungsgemäß
Silica (Sunsphere H-121): sphärisch, mittlere Teilchengröße: 10,5-13,5 µm, Ölabsorption: 30-190 ml/g
Silica (Sunsil-130): "beads", mittlere Teilchengröße: 6-9 µm, Ölabsorption: 0,9-1,3 ml/g
Silica Micro Bead BA-1): sphärisch, mittlere Teilchengröße: 16 µm, Ölabsorption: 50 ml/g
Silica (Valvance Touch 210): runde, poröse beads, mittlere Teiklchengröße: 6-15 µm, Ölabsorption: > 1,3 ml/g

***In-vitro* Sandanhaftung**

50 mg der Test-Emulsion wurden auf PMMA Schönberg Platten (5,0 x 5,0 cm) aufgetragen und gleichmäßig mit einem Fingerling auf der Platte verteilt. Anschließend wird die aufgetragene Beispielrezeptur für 15 min bei Raumtemperatur getrocknet. Danach wurde das Gewicht der getrockneten Platten mit einer Analysenwaage ermittelt. Anschließend wurden die Platten mit feinem Seesand (1.07711.1000 Seesand reinst, der Firma Merck KGaA) im Überschuss übergossen. Durch einmaliges Rutschen der Platten auf einer dafür vorgesehenen Rutschvorrichtung (siehe unten) wurde lose anhaftender Sand mit reproduzierbarer, einheitlicher Kraft entfernt.
Der daraufhin auf der Platte zurückbleibende anhaftende Sand wurde durch Auswiegen ermittelt. Die Sandanhaftung kann mit folgender Gleichung ermittelt werden:

$$\Delta\,(Anhaftung)\,[mg] = m\,(Platte\ mit\ Sand)[mg] - m\,(eingecremte\ Platte)\,[mg]$$

Die Rutschvorrichtung ist eine in Form eines Dreiecks aufgebaute Konstruktion, bei der die Breite der Rutsche 5 cm beträgt. Die Rutschvorrichtung ist eine in Form eines rechtwinkligen Dreiecks aufgebaute Konstruktion, bei der die Breite der Rutsche 5 cm beträgt. Die Ankathete des rechtwinkeligen Dreiecks (= Standfläche der Rutsche) hat eine Länge von 13,5 cm, die Gegenkathete (= Fallhöhe) hat eine Länge von 49 cm. Die Hypotenuse, auf welcher der Rutschvorgang stattfindet, und die Standfläche schließen einen Winkel von 275 ° ein.

Die Versuche wurden je Rezeptur 10x wiederholt und der entsprechende Mittelwert gebildet.

| Ansatz | Platte ohne Sand | Platte mit Sand | Anhaften-der Sand in g | Mittelwert | Standard-abweichung |
|---|---|---|---|---|---|
| MATTSUN30 #17 | 7,146 | 7,159 | 0,013 | | |
| (Sunsphere H-52) | 7,202 | 7,210 | 0,008 | | |
| | 7,174 | 7,190 | 0,016 | | |
| | 7,130 | 7,140 | 0,010 | | |
| | 7,148 | 7,164 | 0,016 | | |
| | 7,124 | 7,165 | 0,041 | | |
| | 7,161 | 7,170 | 0,009 | | |
| | 7,216 | 7,233 | 0,017 | | |
| | 7,131 | 7,146 | 0,015 | | |
| | 7,223 | 7,228 | 0,005 | 0,016 | 0,010 |
| | | | | | |
| MATTSUN30 #19 | 7,213 | 7,244 | 0,031 | | |
| (Valvance Touch 210) | 7,116 | 7,155 | 0,039 | | |
| | 7,157 | 7,193 | 0,036 | | |
| | 7,119 | 7,165 | 0,046 | | |
| | 7,122 | 7,166 | 0,044 | | |
| | 7,167 | 7,203 | 0,036 | | |
| | 7,194 | 7,235 | 0,041 | | |
| | 7,208 | 7,271 | 0,063 | | |
| | 7,140 | 7,189 | 0,049 | | |
| | 7,135 | 7,173 | 0,038 | 0,042 | 0,009 |
| | | | | | |
| MATTSUN30 #21 | 7,142 | 7,236 | 0,094 | | |
| (Sunsil-130) | 7,199 | 7,219 | 0,020 | | |
| | 7,172 | 7,184 | 0,012 | | |
| | 7,131 | 7,192 | 0,061 | | |
| | 7,217 | 7,274 | 0,057 | | |
| | 7,162 | 7,254 | 0,092 | | |
| | 7,122 | 7,183 | 0,061 | | |
| | 7,220 | 7,250 | 0,030 | | |
| | 7,165 | 7,221 | 0,056 | | |
| | 7,141 | 7,180 | 0,039 | 0,052 | 0,028 |
| MATTSUN30 #40 | 7,215 | 7,252 | 0,037 | | |
| (Silica Micro Bead BA-1) | 7,142 | 7,214 | 0,072 | | |
| | 7,128 | 7,139 | 0,011 | | |
| | 7,225 | 7,283 | 0,058 | | |
| | 7,171 | 7,210 | 0,039 | | |
| | 7,143 | 7,165 | 0,022 | | |
| | 7,166 | 7,198 | 0,032 | | |
| | 7,206 | 7,250 | 0,044 | | |
| | 7,129 | 7,149 | 0,020 | | |
| | 7,148 | 7,211 | 0,063 | 0,040 | 0,020 |

7

| MATTSUN30 #41 | 7,269 | 7,350 | 0,081 | | |
| (Sunsphere H-121) | 7,176 | 7,245 | 0,069 | | |
| | 7,153 | 7,220 | 0,067 | | |
| | 7,103 | 7,175 | 0,072 | | |
| | 7,189 | 7,228 | 0,039 | | |
| | 7,181 | 7,235 | 0,054 | | |
| | 7,163 | 7,259 | 0,096 | | |
| | 7,251 | 7,276 | 0,025 | | |
| | 7,157 | 7,187 | 0,03 | | |
| | 7,187 | 7,238 | 0,051 | 0,058 | 0,023 |
| | | | | | |

| Ansatz | anhaftender Sand in mg/cm² | Standardabweichung |
|---|---|---|
| MATTSUN30 #17 | 0,64 | 0,40 |
| MATTSUN30 #19 | 1,69 | 0,36 |
| MATTSUN30 #21 | 2,09 | 1,10 |
| MATTSUN30 #40 | 1,59 | 0,79 |
| MATTSUN30 #41 | 2,34 | 0,91 |

**Beispiele**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Diese Zubereitungen weisen nur eine geringe Sandanhaftung auf.

| INCI | 1 | 2 | 3 | 4 | 5 |
|---|---|---|---|---|---|
| Tocopheryl Acetate | 0,1 | 0,1 | 0,1 | 0,1 | 0,1 |
| Glycyrrhiza Inflata Root Extract | 0,025 | | 0,025 | | 0,025 |
| Ethylhexylglycerin | 0,3 | 0,3 | 0,3 | 0,3 | 0,3 |
| Magnolia Officinalis Bark Extract | | 0,05 | | | |
| Ubiquinone + Aqua | | | 0,03 | 0,03 | |
| Dibutyl Adipate | 2,5 | 2 | 2,5 | | |
| C12-15 Alkyl Benzoate | 3 | 3 | 3 | | 1 |
| Butylene Glycol Dicaprylate/Dicaprate | 3 | 3 | 3 | 4 | 4 |
| Dimethicone | 0,8 | 0,8 | 0,8 | 0,8 | 0,8 |
| Cyclomethicone | | 2 | 1,5 | 1,5 | 2 |
| Sodium Stearoyl Glutamate | 0,3 | 0,3 | 0,3 | 0,4 | 0,4 |
| Silica | 2 | 5 | 0,5 | 2,5 | 3 |
| Tapioca Starch + Aqua | 2 | 1 | 2 | 1,5 | 1,5 |

(fortgesetzt)

| INCI | 1 | 2 | 3 | 4 | 5 |
|---|---|---|---|---|---|
| Parfum | 0,3 | 0,3 | 0,3 | 0,3 | 0,3 |
| Methylpropanediol | 0,5 | 1 | 0,75 | 1 | 1 |
| Glycerin | 1 | 1 | 1 | 1 | 1 |
| Aqua + Sodium Hydroxide | 0,94 | 0,94 | 0,94 | 0,93 | 0,58 |
| Phenoxyethanol | 0,6 | 0,5 | 0,6 | 0,8 | 0,7 |
| Behenyl Alcohol | 1 | 1 | 1,25 | 1 | 1 |
| Cetearyl Alcohol | 1 | 1 | 0,75 | 1 | 1 |
| Carbomer | 0,1 | 0,1 | 0,1 | 0,1 | 0,1 |
| Acrylates/C10-30 Alkyl Acrylate Crosspolymer | 0,15 | 0,15 | 0,15 | 0,15 | 0,15 |
| Xanthan Gum | 0,1 | 0,1 | 0,1 | 0,1 | 0,1 |
| Alcohol Denat. + Aqua | 4,5 | 5 | 4,5 | 4 | 4,5 |
| Aqua | 61,535 | 56,86 | 61,255 | 51,74 | 50,945 |
| Aqua + Trisodium EDTA | 1 | 1 | 1 | 1 | 1 |
| Ethylhexyl Salicylate | 3 | 3,5 | 3 | 4,75 | 4,75 |
| Bis-Ethylhexyloxyphenol Methoxyphenyl Triazine | 2,5 | 2,5 | 2,5 | 3,75 | 4 |
| Ethylhexyl Triazone | 2,5 | 2 | 2,5 | 4 | 3 |
| Butyl Methoxydibenzoylmethane | 3,25 | 2,5 | 3,25 | 4,75 | 4,75 |
| Phenylbenzimidazole Sulfonic Acid | 2 | 2 | 2 | 2 | 1 |
| Diethylamino Hydroxybenzoyl Hexyl Benzoate | | 1 | | | 1 |
| Homosalate | | | | 6,5 | 6 |

## Patentansprüche

1. Verwendung eines sphärischen Silikats mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101) in kosmetischen Zubereitungen zur Reduzierung der Sand anhaftenden Eigenschaften der Zubereitung.

2. Verwendung nach Anspruch 1, **dadurch gekennzeichnet, dass** die Zubereitung mindestens einen im UV-A Bereich absorbierenden organischen UV-Filter enthält.

3. Kosmetische Zubereitung enthaltend

   a) ein sphärisches Silikat mit der INCI Bezeichnung Silica mit einer mittleren Teilchengröße von 4,0 bis 6,0 $\mu$m und einer Ölabsorption von 250 bis 350ml/100g (gemessen nach dem japanischen Industriestandard K-5101)
   b) mindestens einen im UV-A Bereich absorbierenden organischen UV-Filter.

4. Verwendung nach einem der Ansprüche 1 oder 2 oder Zubereitung nach Anspruch 3, **dadurch gekennzeichnet, dass** die Zubereitung das sphärische Silikat in einer Konzentration von 0,5 bis 5 Gewichts-%, bezogen auf das Gesamtgewicht der Zubereitung, enthält.

5. Verwendung oder Zubereitung nach einem der vorhergehenden Ansprüche, **dadurch gekennzeichnet, dass** das sphärische Silikat eine spezifische Oberfläche von 600 bis 800 m$^2$/g (Messmethode: BET mit N$_2$) und ein spezifisches Porenvolumen von 1,4 bis 1,8 ml/g (Messmethode: BET mit N$_2$) aufweist.

6. Verwendung oder Zubereitung nach einem der vorhergehenden Ansprüche, **dadurch gekennzeichnet, dass** der oder die im UV-A Bereich absorbierenden organischen UV-Filter gewählt werden aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine).

7. Verwendung oder Zubereitung nach einem der vorhergehenden Ansprüche, **dadurch gekennzeichnet, dass** die Zubereitung einen oder mehrere weitere UV-Filter gewählt aus der Gruppe der Verbindungen Salicylsäure-2-ethylhexylester (INCI Octyl Salicylate), 3,3,5-Trimethylcyclohexyl 2-hydroxybenzoat (INCI: Homosalate), 2,4,6-Tris-[anilino-(p-carbo-2'-ethyl-1'-hexyloxy)]-1,3,5-triazin (INCI: Ethylhexyl Triazone) und/oder 2-Phenylbenzimidazol-5-sulfonsäuresalze, enthält.

8. Verwendung oder Zubereitung nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), Ethylhexyl-2-cyano-3,3-diphenyl-acrylat (INCI: Octocrylen), Parabenen, Methylisothiazolinon, Chlormethyl-isothiazolinon und DMDM-Hydantoin.

9. Verwendung oder Zubereitung nach einem der vorhergehenden Ansprüche, **dadurch gekennzeichnet, dass** die Zubereitung frei ist von 4-Methoxyzimtsäure-2-ethylhexylester (INCI Octylmethoxycinnamate).

10. Verwendung oder Zubereitung nach einem der vorhergehenden Ansprüche, **dadurch gekennzeichnet, dass** die Zubereitung in Form einer O/W-Emulsion vorliegt.

11. Verwendung oder Zubereitung nach einem der vorhergehenden Ansprüche, **dadurch gekennzeichnet, dass** die Zubereitung Natriumstearylglutamat als Emulgator enthält.

12. Verwendung oder Zubereitung nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, $\beta$-Alanin, Panthenol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze und/oder Licochalcon A enthält.

13. Verwendung oder Zubereitung nach einem der vorhergehenden Ansprüche, **dadurch gekennzeichnet, dass** die Zubereitung ein oder mehrere Alkandiole aus der Gruppe der Verbindungen 1,2-Pentandiol, 1,2-Hexandiol, 1,2-Octandiol, 1,2-Decandiol, 2-Methyl-1,3-propandiol enthält.

14. Verwendung oder Zubereitung nach einem der vorhergehenden Ansprüche, **dadurch gekennzeichnet, dass** die Zubereitung keine Polyethylenglycolether oder Polyethylenglycolester enthält.

15. Verwendung oder Zubereitung nach einem der vorhergehenden Ansprüche, **dadurch gekennzeichnet, dass** die Zubereitung Ethanol, Phenoxyethanol und/oder Ethylhexylglycerin enthält.

**Claims**

1. Use of a spherical silicate with the INCI name Silica, having an average particle size of 4.0 to 6.0 $\mu$m and an oil absorption of 250 to 350 ml/100 g (measured in accordance with the Japanese Industry Standard K-5101), in cosmetic preparations for reducing the sand-adhering properties of the preparation.

2. Use according to Claim 1, **characterized in that** the preparation comprises at least one organic UV filter absorbing in the UVA range.

3. Cosmetic preparation comprising

   a) a spherical silicate with the INCI name Silica, having an average particle size of 4.0 to 6.0 $\mu$m and an oil absorption of 250 to 350 ml/100 g (measured in accordance with the Japanese Industry Standard K-5101)
   b) at least one organic UV filter absorbing in the UVA range.

4. Use according to either of Claims 1 and 2 or preparation according to Claim 3, **characterized in that** the preparation comprises the spherical silicate at a concentration of 0.5 to 5% by weight, based on the total weight of the preparation.

5. Use or preparation according to any of the preceding claims, **characterized in that** the spherical silicate has a specific surface area of 600 to 800 $m^2$/g (measurement method: BET using $N_2$) and a specific pore volume of 1.4 to 1.8 ml/g (measurement method: BET using $N_2$).

6. Use or preparation according to any of the preceding claims, **characterized in that** the organic UV filter(s) absorbing in the UVA range are selected from the group of compounds comprising 4-(tert-butyl)-4'-methoxydibenzoylmethane, hexyl 2-[4-(diethylamino)-2-hydroxybenzoyl]benzoate (INCI: Diethylamino Hydroxybenzoyl Hexyl Benzoate) and/or 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

7. Use or preparation according to any of the preceding claims, **characterized in that** the preparation comprises one or more further UV filters selected from the group of compounds comprising 2-ethylhexyl salicylate (INCI Octyl Salicylate), 3,3,5-trimethylcyclohexyl 2-hydroxybenzoate (INCI: Homosalate), 2,4,6-tris[anilino(p-carbo-2'ethyl-1'-hexyloxy)]-1,3,5-triazine (INCI: Ethylhexyl Triazone) and/or 2-phenylbenzimidazole-5-sulfonic acid salts.

8. Use or preparation according to any of the preceding claims, **characterized in that** the preparation is free of 3-(4-methylbenzylidene)camphor, 2-hydroxy-4-methoxybenzophenone (INCI: Oxybenzone), ethylhexyl 2-cyano-3,3-diphenylacrylate (INCI: Octocrylene), parabens, methylisothiazolinone, chloromethylisothiazolinone and DMDM hydantion.

9. Use or preparation according to any of the preceding claims, **characterized in that** the preparation is free of 2-ethylhexyl 4-methoxycinnamate (INCI: Octyl Methoxycinnamate).

10. Use or preparation according to any of the preceding claims, **characterized in that** the preparation is in the form of an O/W emulsion.

11. Use or preparation according to any of the preceding claims, **characterized in that** the preparation comprises sodium stearoyl glutamate as emulsifier.

12. Use or preparation according to any of the preceding claims, **characterized in that** the preparation comprises one or more compounds selected from the group of compounds comprising alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopheryl acetate, dihydroxyacetone; 8-hexadecene-1,16-dicarboxylic acid, glycerylglucose, (2-hydroxyethyl)urea, vitamin E or derivatives thereof, hyaluronic acid and/or salts thereof and/or licochalcone A.

13. Use or preparation according to any of the preceding claims, **characterized in that** the preparation comprises one or more alkanediols from the group of compounds comprising 1,2-pentanediol, 1,2-hexanediol, 1,2-octanediol, 1,2-decanediol, 2-methyl-1,3-propanediol.

14. Use or preparation according to any of the preceding claims, **characterized in that** the preparation does not comprise any polyethylene glycol ethers or polyethylene glycol esters.

15. Use or preparation according to any of the preceding claims, **characterized in that** the preparation comprises ethanol, phenoxyethanol and/or ethylhexylglycerin.

## Revendications

1. Utilisation d'un silicate sphérique ayant la désignation INCI Silica, présentant une taille de particule moyenne de 4,0 à 6,0 μm et une absorption de l'huile de 250 à 350 ml/100 g (mesurée selon la norme industrielle japonaise K-5101) dans des préparations cosmétiques pour réduire les propriétés d'adhésion au sable de la préparation.

2. Utilisation selon la revendication 1, **caractérisée en ce que** la préparation contient au moins un filtre UV organique absorbant dans la plage des UV-A.

**3.** Préparation cosmétique contenant :

a) un silicate sphérique ayant la désignation INCI Silica, présentant une taille de particule moyenne de 4,0 à 6,0 µm et une absorption de l'huile de 250 à 350 ml/100 g (mesurée selon la norme industrielle japonaise K-5101),
b) au moins un filtre UV organique absorbant dans la plage des UV-A.

**4.** Utilisation selon l'une quelconque des revendications 1 ou 2 ou préparation selon la revendication 3, **caractérisée en ce que** la préparation contient le silicate sphérique en une concentration de 0,5 à 5 % en poids, par rapport au poids total de la préparation.

**5.** Utilisation ou préparation selon l'une quelconque des revendications précédentes, **caractérisée en ce que** le silicate sphérique présente une surface spécifique de 600 à 800 m$^2$/g (méthode de mesure : BET avec N$_2$) et un volume poreux spécifique de 1,4 à 1,8 ml/g (méthode de mesure : BET avec N$_2$).

**6.** Utilisation ou préparation selon l'une quelconque des revendications précédentes, **caractérisée en ce que** le ou les filtres UV organiques absorbant dans la plage des UV-A sont choisis dans le groupe de composés constitué par le 4-(tert.-butyl)-4'-méthoxydibenzoylméthane, le 2-[4-(diéthylamino)-2-hydroxybenzoyl]benzoate d'hexyle (INCI : Diethylamino Hydroxybenzoyl Hexyl Benzoate) et/ou la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine).

**7.** Utilisation ou préparation selon l'une quelconque des revendications précédentes, **caractérisée en ce que** la préparation contient un ou plusieurs filtres UV supplémentaires choisis dans le groupe de composés constitué par l'ester 2-éthylhexylique de l'acide salicylique (INCI : Octyl Salicylate), le 2-hydroxybenzoate de 3,3,5-triméthylcyclohexyle (INCI : Homosalate), la 2,4,6-tris-[anilino-(p-carbo-2'-éthyl-1'-hexyloxy)]-1,3,5-triazine (INCI : Ethylhexyl Triazone) et/ou les sels de l'acide 2-phénylbenzimidazole-5-sulfonique.

**8.** Utilisation ou préparation selon l'une quelconque des revendications précédentes, **caractérisée en ce que** la préparation est exempte de 3-(4-méthylbenzylidène)-camphre, de 2-hydroxy-4-méthoxybenzophénone (INCI : Oxybenzon), d'acrylate d'éthylhexyl-2-cyano-3,3-diphényle (INCI : Octocrylen), de parabènes, de méthylisothiazolinone, de chlorométhyl-isothiazolinone et de DMDM-hydantoïne.

**9.** Utilisation ou préparation selon l'une quelconque des revendications précédentes, **caractérisée en ce que** la préparation est exempte d'ester 2-éthylhexylique de l'acide 4-méthoxycinnamique (INCI : Octylmethoxycinnamate).

**10.** Utilisation ou préparation selon l'une quelconque des revendications précédentes, **caractérisée en ce que** la préparation se présente sous la forme d'une émulsion H/E.

**11.** Utilisation ou préparation selon l'une quelconque des revendications précédentes, **caractérisée en ce que** la préparation contient du glutamate de stéaryle sodique en tant qu'émulsifiant.

**12.** Utilisation ou préparation selon l'une quelconque des revendications précédentes, **caractérisée en ce que** la préparation contient un ou plusieurs composés choisis dans le groupe de composés constitué par l'acide alpha-liponique, l'acide folique, le phytoène, la D-biotine, la coenzyme Q10, l'alpha-glucosylrutine, la carnitine, la carnosine, les isoflavonoïdes naturels et/ou synthétiques, les flavonoïdes, la créatine, la créatinine, la taurine, la β-alanine, le panthénol, le magnolol, l'honokiol, l'acétate de tocophéryle, la dihydroxyacétone, l'acide 8-hexadécène-1,16-dicarboxylique, le glycérylglycose, la (2-hydroxyéthyl)urée, la vitamine E ou ses dérivés, l'acide hyaluronique et/ou ses sels et/ou la licochalcone A.

**13.** Utilisation ou préparation selon l'une quelconque des revendications précédentes, **caractérisée en ce que** la préparation contient un ou plusieurs alcanediols du groupe de composés constitué par le 1,2-pentanediol, le 1,2-hexanediol, le 1,2-octanediol, le 1,2-décanediol, le 2-méthyl-1,3-propanediol.

**14.** Utilisation ou préparation selon l'une quelconque des revendications précédentes, **caractérisée en ce que** la préparation ne contient pas d'éthers de polyéthylène glycol ou d'esters de polyéthylène glycol.

**15.** Utilisation ou préparation selon l'une quelconque des revendications précédentes, **caractérisée en ce que** la préparation contient de l'éthanol, du phénoxyéthanol et/ou de l'éthylhexylglycérine.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3093007 A1

**In der Beschreibung aufgeführte Nicht-Patentliteratur**

- *CHEMICAL ABSTRACTS,* 178463-23-5

- *CHEMICAL ABSTRACTS,* 7631-86-9